# EUROPEAN PATENT SPECIFICATION

(11) **EP 2 766 038 B1**
(45) Date of publication and mention of the grant of the patent: **02.03.2016**
(21) Application number: 13785956.7
(22) Date of filing: 03.09.2013
(51) Int. Cl.: A61K 39/12

(54) **CCHFV VACCINE**
CCHFV-IMPFSTOFF
VACCIN CONTRE LE CCHFV

(30) Priority: 06.09.2012 TR 201210186
(43) Date of publication of application: 20.08.2014
(73) Proprietor: Firat Üniversitesi, 23119 Elazig (TR)
(72) Inventor: ÖZDARENDELI, Aykut, Kayseri (TR); ÇANAKOGLU, Nurettin, Elazig (TR); BERBER, Engin, Elazig (TR); AKTAS, Münir, Elazig (TR); KALKAN, Ahmet, Trabzon (TR)
(74) Representative: Bulut, Pinar
(86) International application number: PCT/TR2013/000284
(87) International publication number: WO 2014/039021

(56) References cited:
- KESHTKAR-JAHROMI MARYAM ET AL: "Crimean-Congo hemorrhagic fever: Current and future prospects of vaccines and therapies", ANTIVIRAL RESEARCH, vol. 90, no. 2, May 2011 (2011-05), pages 85-92, XP002718177,
- OZDARENDELI AYKUT ET AL: "The complete genome analysis of Crimean-Congo hemorrhagic fever virus isolated in Turkey", VIRUS RESEARCH, vol. 147, no. 2, February 2010 (2010-02), pages 288-293, XP002718178, ISSN: 0168-1702

## Description

### Technical Field

Crimean Congo Hemorrhagic Fever (CCHF) virus is a zoonotic agent belongs to the Nairovirus genus of the family Bunyaviridae. Currently, no protective vaccine is available against CCHF. In order to develop a vaccine against CCHF which has become one of the most important public health problems in Turkey since 2002, a vaccine to be used against CCHF in humans, being prepared in the Vero E6 cell culture, purified and inactivated with formalin is developed.

### Prior Art

The vaccines produced by the preparation technique used by us, i.e. the vaccines produced in cell culture, purified and used by being inactivated are available in the world. In 2006, the CCHF virus Kelkit06 strain (reference) isolated from a CCHF patient in the province of Gumushane (Kelkit) is used as the master virus in the preparation of the inactivated vaccine. In the world, there is no vaccine against CCHF. However, in 1971, the vaccine prepared in the former Russia's Rostov region in the brains of suckling mice by being inactivated with formalin, the inactivated vaccine prepared in the same manner and administered to 1500 people, is tried in 583 volunteers in Bulgaria. In addition, since the vaccine preparation studies against the CCHF virus was conducted long time ago with very limited working conditions; relevant satisfactory scientific data on the subject matter does not exist. In 2005, in a DNA immunization study conducted with mice using the M gene of the CCHF virus, only 50% of immunized mice developed neutralizing antibodies, however, challenge experiments could not be realized due to the absence of an animal model. The number of vaccine studies conducted against important zoonotic agents comprised in this virus family has especially increased in recent years. The leading vaccine development work is against the hantaviruses causing high mortality rates in humans and classified as biological weapons. Vaccines against Hemorrhagic Fever with Renal Syndrome (HFRS) prepared in the brains of rodents as well as cell cultures and tested in humans in Asia are available. The inactivated vaccines prepared with rodent brains has not found use in the Western countries. In Korea, the commercial Hantavax vaccine prepared in the brains of the suckling mice has been used in humans for over 10 years. The Hantavax vaccine inactivated with formalin and adjuvanted with aluminum hydroxide is administered with two doses at a 1-month interval and then a booster dose is administered after 12 months. Although formation of antibodies was observed as a result of the ELISA tests applied to vaccinated people, formation of neutralizing antibodies in only half of the vaccinated people is reported. Field studies with Hantavax vaccine is not carried out and case control studies are not considered sufficient for generation of statistical data. In China, inactivated vaccines are prepared by growing the Hantaan virus (HTNV) and Seoul virus (SEOV) in a cell culture system and compared with the HTNV vaccines prepared in the brains of suckling mice in terms of immunity. In the study carried out with approximately 100,000 people, it is observed that the SEOV vaccine forms neutralizing antibodies in 80% of vaccinated people when it is administered in 3 doses, while it forms neutralizing antibodies in 50% of vaccinated people when it is administered in 2 doses. In another study, a bivalent vaccine is used and said vaccine is produced in the SEOV and HTNV cell cultures, inactivated with formalin, purified by column chromatography and adjuvanted with aluminum hydroxide. It is administered to people in 2 doses at a two week interval and neutralizing antibodies are formed in 93% of people administered therewith. In recent years, safer molecular approaches have been investigated in vaccine development in order to eliminate the disadvantages of vaccines prepared in cell cultures or mice brains. M and S gene products of the HTNV are produced by using recombinant vaccinia virus systems and tested in 16 volunteers. No side effects are observed and formation of neutralizing antibodies is encountered only in high dose administered volunteers. In the phase 2 study conducted using the same system, formation of neutralizing antibodies is observed in 72% of the high dose administered recombinant vaccine volunteers. DNA vaccine is developed as another molecular hantavirus vaccine approach and phase 1 studies in volunteers are still ongoing. Rift Valley Fever (RVF) virus is one of the most important zoonoses of the Bunyaviridae family. The formalin-inactivated vaccine NDBR 103 is prepared by producing the Entebbe strain of RVF in the primary monkey kidney cells by 184 mouse brain passages and tested in 500 volunteers in 1977. In later years, FRhL-2 cells are used in the preparation of formalin-inactivated vaccine TSI-GSD 200 and safer laboratory conditions are created. The TSI-GSD 200 vaccine is administered to the volunteers in 3 doses at days 0, 7, and 28 and a neutralizing titer of 1:40 or higher is obtained in 90% of the vaccinated volunteers. In addition, there are formalin-inactivated vaccine applications prepared from the pantropic strain of RVF used in veterinary medicine for vaccination of the sheep. Live attenuated RVF vaccines have been investigated for many years. These vaccines are MP-12 live vaccine strains having the potential to be used in both humans and animals. 12 passages in the presence of 5-fluorouracyl (5FU) is carried out and pathogenicity thereof is eliminated by obtaining a temperature sensitive mutant virus.

### REFERENCES

1- Elliot RM., Bouloy M., Calisher CH., Goldbach R., Moyer JT., Nichol ST., Petterson P., Schmaljohn, CS., Family *Bunyaviridae.* In: van regenmortel, MHV., Fauquet, CM., Bishop, DHL., Carstens EB., Estes, MK., Lemon S., Maniloff J., Mayo MA., McGeogch D., Pringle CR., Wickner RB., Virus Taxonomy. Seventh Report International Committee Virus Taxonomy. Seventh Report International Committee of Viruses. Academic Press, San Diego, CA, 599-621, (1996).
2- Elliot RM., Molecular Biology of Bunyaviruses. Jounal of General Virology, 71, 501-522, (1990).
3- Ikegami T., Makino S. Rift Valley Fever Vaccines. Vaccine, 27, D61-D64, (2009).
4- Schmaljohn C., Vaccines for Hantaviruses. Vaccine, 27, D61-D64, (2009).
5- Spik K., Shurtleff A., McElroy AK., Guttieri CM., Hooper JW., Schmaljohn C. Immunogenicity of combination DNA vaccines for Rift Valley fever virus, tick-borne encephalitis virus, Hantaan virus, and Crimean Congo hemorrhagic fever virus. Vaccine, 24, 4657-4666, (2006).
6- Tkanchenko EA., Butenko AM., Badalov ME., Zavodov TI., Chumakov MP., Investigation of the immunogenic activity of killed brain vaccine against Crimean hemorrhagic fever, Tr. Inst. Polio. Virusn. Entsegalitov Akad. Med. Nauk SSSR, 19, 119-129, (1971).
7- Vasilenko SM., Results of the investigation on etiology, epidemiologic features and the specific prophylactic of Crimean hemorrhagic fever (CHF) in Bulgaria, In: Abstr. Inv. Pap. 9. Int. Congr. Trop. Med. Malar., Athens, October 1973, Vol: I, 32-33.
4- Whitehouse CA., Crimean Congo Hemorrhagic Fever Virus., Antiviral research, 64, 145, 157-160, (2004).
8- Zoonozlar., Ed. Doǧanay M, Altinta 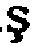 M. Bunyavirüslerin Neden Oldugu Zoonozlar Ergönül Ö, Ozdarendeli A. p. 475-510. Bilimsel Tip Yayinevi. Ankara (2009).
g- Keshtkar-Jahromi et al., Crimean-Congo hemorrhagic fever: Current and future prospects of vaccines and therapies, Antiviral Research 90:85-92(2011)

### Objects of the Invention

Since 2002, CCHF is one of the major health problems of our country. The mortality in humans ranges from 5-30%. The disease is seen not only in our country, but also in some countries in the Middle East, Asia, Africa and Eastern Europe. However, a licensed vaccine developed against the CCHF is not currently available in the world. In this context, the inactivated CCHF vaccine developed by using cell cultures will be one of the most important preventive measures against said disease resulting in death.

### Description of the Figures

Figure 1: Determination of titer of the CCHF Kelkit06 isolate. VK: virus control, HK: cell control.
Figure 2: Demonstration of the CCHF virus antigens before purification (A) and after purification (B) by staining with SDS-PAGE, demonstration of the proteins of the CCHF virus by western blot using polyclonal rabbit serum (C).
Figure 3: Neutralizing antibody titers of the mice vaccinated with the cell culture based inactivated vaccine developed against CCHF after the vaccination 2 and 3.

### Description of the Invention

Viral antigens are obtained by producing the Kelkit06 strain of the CCHF virus isolated in our country in the Vero E6 cells. Concentration, purification and inactivation of the CCHF antigens are realized. Then, it is adjuvanted with ALUM (aluminum hydroxide) adjuvant and cell culture based inactivated CCHF vaccine is developed by the addition of 0.001% of thimerosal into the final vaccine formulation. The vaccine tested in the IFNAR mice demonstrates formation of high neutralizing antibody titer and protection.

### Detailed description of the present invention is given below:

### Production of the Kelkit06 strain of the CCHF virus:

For the Kelkit06 strain of the CCHF virus to be used in the production of the vaccine antigens, passage of 80% confluent 5x10⁶ Vero E6 cells in 20 ml of cell growth medium in a 175 cm² flask (Coming, Cat.CLS431079) is carried out 16 to 24 hours in advance. 16 hours after the passage, the medium used for growing the cells is discarded prior to performing the infection and culturing is carried out by means of an adsorption based method by diluting the virus produced in the mouse brain with a known titer of 0.01 MOI in 5 ml DMEM F-12 in each flask provided with mouse brain stocks having determined virus titers. Flasks are shaken with 10 minute intervals in 5% CO₂ incubator at 37°C and infection is realized for 1 hour. After the infection is performed, the inoculum is discarded and 20 ml of virus growth medium is added (3% FBS, 1% antibiotic, DMEM F-12). The flasks are placed again in the 5% CO₂ incubator at 37°C and then observed daily under a microscope until 70% of the cells decomposes. The flasks provided with virus growth are stored at -80°C.

### Titration test of the CCHF virus Kelkit06 strain produced in VERO E6 cells (pseudo-plaque test):

One day before, by removing Vero E6 cells with tripsin (Sigma Cat.T4049), these cells (20x10⁴/1 ml for each well) are placed into 24 welled plate (Costar Cat.3524) within cell production medium (10% FBS (Sigma Cat.F2442), 1% antibiotic (Sigma Cat. A5955), DMEM F-12 HAM (Sigma Cat.D8900)). Seven Centrifuge tubes (USA Scientific Cat.1615-5500) were marked and 0.9 ml DMEM F-12 was added to all tubes as a dilution solution. 0.1 ml of the virus stock, the titre of which will be calculated is added to the first tube and vortexed. 0.1 ml is taken from the first tube and and added to the second tube, thus the serial dilution of the virus is obtained from 10⁻¹ to 10⁻⁷ in log 10. The culturing medium in the cells which was poured one day before was collected and 0.2 ml diluted virus was poured onto the wells four times for each dilution onto the monolayer cells beginning first with the cell control, from the highest dilution to the lowest dilution. 0.2 ml of the diluted virus which was diluted as virus control was poured to two wells and solely 0.2 ml of the dilution solution was added to two wells as cell control. The plate was placed into the incubator (37°C, 5% CO₂) and inoculation bound to absorption was performed for one hour with 15 minutes shaking. At the end of the infection, the inoculum in the wells was discarded and 1% carboxy methyl cellulose heated in water bath at 37°C (2% carboxy methyl cellulose (Sigma Cat.9481) was dissolved in dH₂O, in a 300°C heater (Fissher Scientific Cat.11-300-49SHP) by being mixed with 500 rpm and was prepared by being autoclaved at 121°C for 15 minutes) was mixed with DMEM F-12 prepared at 2X concentration at 37°C with a proportion of 1:1 (3% FBS and 1% antibiotic was added) and then, 1 ml of this mixture was added to each well and plate was placed into the incubator at 37°C, 5% CO₂.

### Visualization of the test:

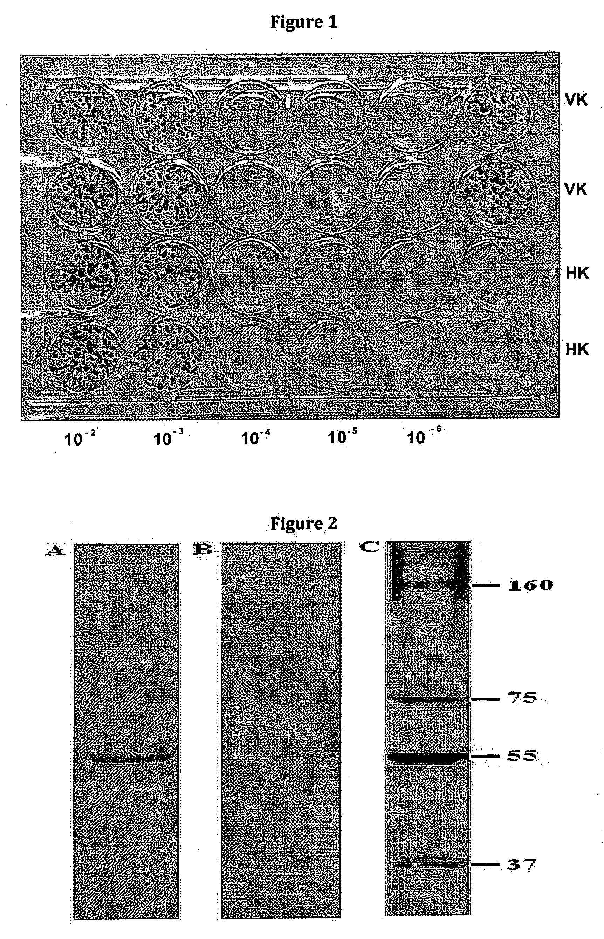

In the fourth day after the infection 0.5 ml 10% neutral buffer formalin solution (4 gr sodium phosphate monobasic (Merck, K91348245 738), 6.5 gr sodium phosphate dibasic (Sigma, S0876), 100 ml, 37% formaldehyde (Sigma Cat.F8775), 900 ml dH₂O, pH 6.8) was added to the plate in the sterile cabin and the cells were fixed in room temperature for 20 minutes. At the end of the incubation, the solution in the wells was discarded and the cells were washed with 1 ml PBS (Amresco, Cat.E404-200 TABS). The cells were permeabilized by adding 0.5 ml of permeabilization solution (PBS comprising 0,1% Triton X-100 (Sigma, Cat.T9284)) to the wells in the shaker (IKA KS260, Cat. 2980300) for four times at 50 rpm at room temperature for 5 minutes. After the last permeabilization process, the cell are blocked by being incubated in the shaker at 50 rpm for 30 minutes at room temperature after adding 0.5 ml 5% skimmed milk powder (Pinar Süt Tozu) prepared in PBS into each well. The cells are washed with 1ml PBS after being blocked. Polyclonal rat serum was diluted in TBST (10mM Tris-HCl pH 8.0 (Sigma, Cat.T3253), 150 mM NaCl (Merck, Cat.K92033000 546), 0,1% Tween-20 (Merck, Cat.S4662084636) içeren dH₂O) in a ratio of 1/1500 and incubated for 1 hour at 50 rpm in the shaker at room temperature by adding 0.2 ml to each well. After being washed three times with 10 minutes intervals with TBST, the wells are incubated for one hour, at room temperature, in a 50 rpm shaker with Goat Anti-Mouse β-gal conjugate (Southern Biotech, Cat.1010-06) which is diluted to a ratio of 1/1500 in TBST. The cells are washed again with TBST three times with ten minutes intervals and then, 0.2 ml substrate solution (5mM MgCl (Merck, Cat.S4845833 730) is diluted in PBS in a ratio of 1/300 from X-Gal (Sigma, Cat.B4252) prepared as 50mg/ml with 99% dimethylformamide (Sigma, N6876), 1/300 from NBT (Sigma, Cat.N6876) prepared as 83mg/ml with 70% dimethylformamide)) is added to each well and incubated at 37°C for 15-30 minutes. The wells are washed with dH₂O and the formed focuses are counted with the naked eye after the infected cells become more clear (Figure 1). The formula average counted focus number/inoculum amount X dilution ratio = ppu/ml is used to calculate the titer.

### The concentration of Kelkit06 strain of the CCHF virus:

The flasks which were stored at -80°C are thawed and then, the virus containing medium is placed into 50 ml tubes (Falcon, Cat.2070) and the cell pieces are discarded by centrifugation at +4°C, at 3000 rpm (Nüve, NF800R) for 30 minutes. 30 ml % 23 NaCl which is the 15 % of (by volume) 200 ml medium filled to glass volumetric flasks is mixed at +4°C, at 100 rpm for 16 hours after the slow addition of 23 ml (Promega, Cat.V3011) which is 10% of the 230ml final volume. Virus PEG mixture which is filled into SW28 ultracentrifuge tubes (38 ml to each one) (Beckman, Polyallomer Cat.326823) is centrifuged for 30 minutes at +4°C, at 12500 rpm in an ultracentrifuge (Beckman, Optima L-70K). The first formed supernatant is discarded and the pellets formed are dissolved with 500 µl 1X TEN buffer (10 X 0.1 TrisCl (pH 8.0), 0.001 M EDTA (pH 8.0), 1 M NaCl) for each tube and then combined. After centrifugation at +4°C, at 5500 rpm for 20 minutes, the second supernatant formed is collected. The virus (Beckman, Ultraclear Cat.344059) precipitated with PEG is completed to 12ml in the SW 41 tubes by adding TEN buffer and centrifuged at +4°C, at 24000 rpm for 2 hours. After the supernatant is discarded, the pellet is dissolved with 1 ml TEN buffer without being broken. All of these processes are realized in an ice bucket, in a cabinet with sterile air circulation.

### Lowry Test:

Lowry test is used for measuring the Protein amounts. To this end, 0.1, 0.3, 0.5, 0.75, 1, 2 and 3 mg/ml BSA (Promega, DM2411) standards are prepared. Standards and 5 µl of the samples to be measured are placed into the 96 welled microplates (Falcon, Cat.353077). 20 µl Reagent A (Biorad Dc Protein Assay, Cat.500-0113) and 200 µl Reagent B (Biorad Dc Protein Assay, Cat.500-0114) are added onto the wells and kept in room temperature for 15 minutes by being pipetted. The amount of proteins in the samples is determined qualitatively by comparing the color changes in the wells with the standards.

### Preparation of the Sucrose Layers:

Sucrose in the amounts of %20, %30, %40, %50 and %60 w/w (Sigma, Cat.S7903) are weighted and dissolved in TEN buffer. The solutions are freshly prepared by being filtered through 0.22 µm (Sartorius, Cat.16534) filter. Beginning from the bottom of the SW 41 tube, 60%, 50%, 40%, 30% and 20% sucrose solutions are added in order, with 12 G injector, with 1 rpm speed, with a peristaltic pump (GE Healthcare, Pump P-1) slowly, from the side of the tube, one on another, with layers of 2ml. After being kept at room temperature for three hours, 1 ml of the virus to be purified is slowly added onto the gradient layers. It is centrifuged at 24000 rpm, at +4°C'de for 18 hours.

### Separation of the fractions:

Purification is carried out by the gradient collector (GE Healthcare, Frac 920) device connected to the peristaltic pump. The infusion hose is connected to the peristaltic pump and to one end of the hose, a 21 G injector and to the other end; a 10 cm injector is connected. The 10 cm injector is gently plunged from the middle of the SW 41 tube until it reaches to the bottom of the tube without disturbing the gradients. The end with 21 G injector is connected to its own place at the arm of the collector device. 1,5 ml centrifuge tubes are placed onto the holes located at the body of the collector and the peristaltic pump is operated at 3 rpm and the first fraction has arrived to the end of the collector. Then, the device is programmed to divide into fractions of 1 ml. The device is operated and the fractions are separated into marked 1 ml tubes. After collecting the last fraction, the hose was prepared for the next tube by being washed with a 10 ml TEN buffer. The collected fractions are kept at +4°C until they are tested with western blot.

### SDS Gel Preparation:

After the system set up of Mini-PROTEAN Tetra Cell (Biorad, Cat.165-8000), 10% dissolving gel is prepared, (it was prepared by mixing 1.9ml dH₂O, 1.7ml 30% acrylamide (Merck, Cat.800830) with 1 gr bisacrylamide (Promega, Cat.V3143), dissolving it and then, sieving it through 0,45 um filter) 1.3 ml 1.5 M Tris (pH 8.8) (Merck, Cat.K37266187 725), 0.05 ml 10% SDS (Biorad, Cat.161-0302), 0.05 ml 10% ammonium persulfate (Applichem, Cat.A2941,0250), 0.002 ml TEMED (Merck, Cat. M1107320 100) and poured between the glass plates, and 1 ml dH₂O is added onto the gel by the help of pipette. The gel was kept 15 minutes for drying and then, the water on the gel is removed and the 5% batching gel is prepared (prepared by mixing 1.4 ml dH₂O, 0.33 ml 30% acrylamide, 0.25 ml 1.0 M Tris (pH 6.8), 0.02 ml 10% SDS, 0.02 ml 10% ammonium persulfate, 0.002 ml TEMED) and the dissolution is placed onto the gel. Comb (Biorad, Cat. 165-3354) is attached and left in room temperature for freezing. The samples to be tested are mixed with Laemli Sample Buffer (Biorad, Cat.161-0737) (before being used 50 µl BME (2-Mercaptoethanol) (Biorad, Cat.161-0710) is added to 950 µl) with a ratio of 1:1 and denaturated in boiling water for 5 minutes. The glass plate in which the prepared gel is present is placed into the gel developing chamber and right after the removal of the comb, 30 µl of the samples which were taken to the ice box are loaded to the wells. The power source (Biorad, Cat.164-5052) is connected to the tank and the samples are left for migrating for 1.5 hours, in Tris/Glycine migration buffer (25 mM Tris (Merck, Cat.K37266187 725), 192 mM glycine (Merck, Cat.K40427490 940), 0.1% SDS, pH 8.3) at 150 volt.

### SDS Gel Staining:

In order to visualize the samples in the gel in which the proteins migrate, the R-250 is stained for 4 hours in the shaker at room temperature at 90 rpm speed after being placed into the stain solution (Biorad, Cat.161-0436). The stain is discarded and in order to remove the stain residues which do not bind to proteins, it is washed with the gel stain washing solution (30% methanol (Sigma, Cat.34885), 60% dH₂O, 10% glacier acetic acid (Merck, Cat.K40973456 014) with 15 minutes intervals on the shaker at room temperature for one hour. As soon as the bands visually become clear, the washing is ceased and the gel is placed into dH₂O cup.

### Western Blot:

In order to transfer the proteins to transfer membrane (Millipore, Immobilon-NY+ Cat.INYC00010), a membrane in the size of gel is cut, its active surface is marked and it is activated in the methanole. The gel that is removed from the glass plate, sponges (Biorad, Cat.1703933) and blotting paper (Biorad, Cat.1703932) are wetted in the transfer buffer (3.3 gr Tris, 14,4 gr Glycine, 100 ml methanole, 900 ml dH₂O) and the lid of the cassette is closed after placing the sponge, blotting paper, membrane, gel, blotting paper and sponge respectively onto the cassette within the buffer. After the cassette is placed into the transfer system apparatus, the system is filled with system transfer buffer and ice battery and fish are placed into it. After the system is placed onto the mixer, it is mixed at 100 rpm. After the power supply is connected to the transfer system, the transfer was realized at 100 volt for 60 minutes. After the transfer process is completed, the membrane is taken out of the cassette and it is blocked in PBST-20 (PBS, 0.1% Tween-20 (Merck, Cat.S4662084), in 5% skimmed milk powder for 30 minutes. After the blocking process, it is incubated at 90 rpm, at room temperature for one hour in the polyclonal rabbit serum prepared in the 10ml 5% skimmed milk powder with a ratio of 1/2000. The primary serum is poured out and the membrane is washed with PBST-20 three times with 10 minutes intervals. As a conjugate, Goat Anti Rabbit HRP serum (Southern Biotech, Cat.4030-05) is diluted in 10ml 5% skimmed milk powder to a ratio of 1/2000 and poured onto the membrane and incubated in the shaker at 90 rpm for one hour at room temperature. The conjugate is poured out and the membrane is washed 3 times with PBST-20 with 10 minutes intervals. The visualization substrate (Thermo Pierce, Cat.32106) is dispersed on the membrane by being mixed at a ratio of 1:1 and it is provided to spread all over the membrane for 5 minutes. The membrane is taken out of this solution, a stretch film is covered onto it, it is placed into a visualization cassette (Fisher, Cat.FBCS 810), a film (Sigma, Cat.F5763) is covered onto it in a dark room and it is incubated for 5 minutes. The film is taken out of the cassette, kept in the developer solution (Sigma, Cat.P7042) for 3 minutes, washed in the dH₂O cup and then kept in the fixer solution (Sigma, Cat.P7067) for 2 minutes. After being washed in the water cup, the developed film is evaluated.

### The purification of Kelkit06 strain of the CCHF virus:

In Western blot test, the fractions rich in virus proteins are gathered and TEN buffer is added until the total volume becomes 12 ml. It is centrifuged at 24000 rpm, at +4°C'de for 2 hours in SW 41 tubes. The supernatant is discarded, 1 ml TEN buffer is added to the pellet formed under the tube and its dissolution is aided by the help of finger power at +4 °C. The purification of the virus is checked by SDS Gel staining (Figure 2).

### The inactivation of Kelkit0b strain of the CCHF virus:

For the virus inactivation, 37% formaldehyde is added to the purified virus in an amount of 0.05 % (it is used with a ratio of 1:50 after being diluted in PBS with a ratio of 1:40) and inactivated in room temperature for 96 hours. To neutralize the formaldehyde, 39% sodium bisulfate (Merck, Cat.S6058956 018) is added to the inactivated antigen in an amount of 3.9%. To remove the residues of formaldehyde and sodium bisulfate, 20.000 MWCO dialysis cassette (Thermo, Cat.66003) is used. PBS is placed into the beaker in an amount of 500 fold of the virus volume and then, the dialysis cassette is wetted for 30 seconds and vaccine antigen is transferred into it by a 21 G injector. The cassette is immersed into the PBS and placed on the mixer. The PBS is changed twice at room temperature with two hour intervals and finally the virus is dialyzed against PBS for one night at room temperature.
The inactivated vaccine is taken out of the cassettes and it is stored at -20°C after measuring its protein quantity with Lowry test.

### Trial of CCHF Inactive Vaccine in IFNAR (- -) Knockout Mouse:

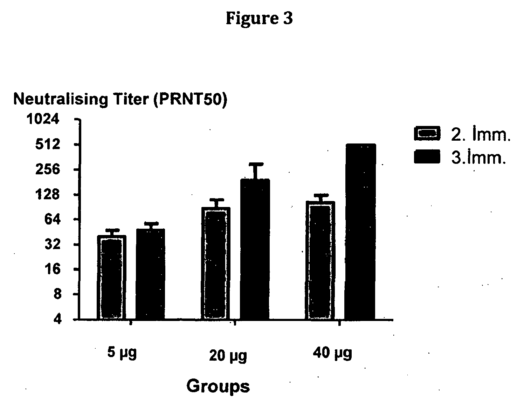

To this end, 6-8 weeks old female IFNAR (- -) knockout mice (Interferon alpha and beta genes removed) are used. Four groups (5 µgr, 20 µgr, 40 µgr inactive antigen and control group (just PBS and/or PBS+adjuvant)) each comprising 5 mice are formed. The antigen specified as 5 µgr, 20 µgr and 40 µgr is diluted in 200 µl PBS. For the control group, only 200 µl PBS is used and the tubes are placed in the vortex device. 660 µgr Imject Alum aluminum hydroxide (Thermo, Cat.77161) is added to the tubes drop by drop at 50rpm speed. The vaccine antigen is adjuvanted by being vortexed at room temperature for 30 minutes. The mice are handled tightly with one hand from their back and neck and with the help of the other hand, they are immunized using an insulin injection from the intraperitoneal route. Immunizations are applied 3 times with three weeks intervals. Blood samples are taken with one week intervals, one day before the immunizations and their neutralizations titers are determined (Figure 3). Two weeks after the third immunization, 1000 pseudo-plaque forming CCHF is challenged with Kelkit06 isolate. The mice in the control group are died after 56-76 hours after the challenge. In 5µgr vaccine group, two mice out of five were dead in 156-176 hours and the other three mice survived, meaning that this vaccine provided 60% protection. In the 20µgr vaccine group (the 2nd vaccine group), one mouse has died in 185th hour and the other four mice survived, meaning that this vaccine group provided 80% protection. In the 40µgr vaccine group (the 3rd vaccine group), one mouse has died in 213rd hour and the other four mice survived, meaning that this vaccine group provided 80% protection.

### The industrial practice of the invention

In the case that the inactive vaccine developed against CCHF is used in humans, the vaccine may be produced in industrial scale by private sector or by Turkish Republic, Ministry of Health. Thus, an important step will be taken as a protective measure against CCHF which is a major health problem in our country. Additionally, it will also be possible to market the vaccine to the other countries in which CCHF is seen. In this sense, with the biotechnological vaccine production and marketing, our country will give acceleration to the industry and become a model.

## Claims

1. Vaccine to prevent and treat Crimean Congo hemorrhagic fever (CCHF) comprising viral antigens, wherein the Kelkit06 strain of Crimean Congo hemorrhagic fever (CCHF) virus is used to obtain said viral antigens.

2. Vaccine according to Claim 1, wherein the Kelkit06 strain is produced in cell culture, purified and inactivated.

3. Vaccine according to Claim 1, wherein it is adjuvanted with Alumiuum Hydroxide (ALUM) adjuvant.

4. Vaccine according to Claim 1, wherein it comprises thimerosal.

5. Vaccine according to Claim 4, wherein the amount of thimerosal is 0.001% (w/w) by weight.

## Patentansprüche

1. Impfstoff zur Vorbeugung und Behandlung Krim-Kongo hämorrhagisches Fieber (CCHF), das von viralen Antigenen, in denen der Kelkit06 Stamm von Krim-Kongo hämorrhagisches Fieber (CCHF) ein Virus ist, wird verwendet, um die die virale Antigene zu erhalten.

2. Impfstoff nach Anspruch 1, in dem der Kelkit06 Stamm in der Zellkultur produziert, in dem er gereinigt und inaktiviert wird.

3. Impfstoff nach Anspruch 1, der mit Aluminiumhydroxid (Alaun) Adjuvans adjuvantiert ist.

4. Impfstoff nach Anspruch 1, der darin Thimerosal umfaßt.

5. Impfstoff nach Anspruch 4, der als Gewichtsmenge 0,001% (w/w) Thiomersal beträgt.

## Revendications

1. Le vaccin pour prévenir et traiter la fièvre hémorragique de Crimée-Congo (FHCC) comportant des antigènes viraux où la souche Kelkit06 du virus de fièvre hémorragique de Crimée-Congo (FHCC) est utilisée pour obtenir lesdits antigènes viraux.

2. Le vaccin selon la revendication 1, dans lequel est produite la souche Kelkit06 dans la culture cellulaire purifiée et inactivée.

3. Le vaccin selon la revendication 1, dans lequel on a ajouté un adjuvant d'hydroxyde d'aluminium (ALUM).

4. Le vaccin selon la revendication 1, dans lequel ce dernier comprend du thimérosal.

5. Le vaccin selon la revendication 4, dans lequel le montant du thimérosal est de l'ordre de 0,001% (p/p) en poids.
